# FASCICULE DE BREVET EUROPEEN

(11) **EP 3 552 648 B1**
(45) Date de publication et mention de la délivrance du brevet: **17.02.2021**
(21) Numéro de dépôt: 19166861.5
(22) Date de dépôt: 02.04.2019
(51) Int. Cl.: A61M 16/00, A61M 16/20

(54) **APPAREIL DE VENTILATION ARTIFICIELLE À SÉCURITÉ AMÉLIORÉE**
GERÄT ZUR KÜNSTLICHEN BEATMUNG MIT VERBESSERTER SICHERHEIT
ARTIFICIAL VENTILATION APPARATUS WITH IMPROVED SAFETY

(30) Priorité: 09.04.2018 FR 1853081
(43) Date de publication de la demande: 16.10.2019
(73) Titulaire: Air Liquide Medical Systems, 92160 Antony (FR)
(72) Inventeur: FOURNIER, Maxence, 92160 ANTONY (FR); BLETON, Héloïse, 92160 ANTONY (FR); LIBARDI, Michael, 78350 Les Loges-En-Josas (FR)
(74) Mandataire: Air Liquide

(56) Documents cités:
- WO-A1-2012/032434
- WO-A1-2017/149532
- US-A1- 2010 078 024
- US-A1- 2017 368 277

## Description

L'invention concerne un appareil de ventilation artificielle offrant un niveau de sécurité accru pour le patient, que ce soit en ventilation sans fuite ou en ventilation à fuite, notamment en facilitant l'évacuation des gaz riches en CO₂ expirés par le patient en cas de dysfonctionnement.

Un appareil de ventilation artificielle, aussi appelé appareil d'assistance respiratoire ou ventilatoire ou plus simplement ventilateur médical, permet d'assurer une ventilation d'un patient souffrant de troubles respiratoires.

Un tel appareil de ventilation artificielle comprend un circuit de gaz, aussi appelé circuit patient, pour véhiculer le gaz, tel de l'air ou de l'air enrichi en oxygène, depuis une source de gaz respiratoire, telle une micro-soufflante, encore appelée turbine ou compresseur, jusqu'aux voies aériennes du patient, le gaz lui étant administré au moyen d'une interface respiratoire, tel un masque respiratoire ou une sonde d'intubation ou analogue.

Une filtration de l'air est préférentiellement opérée au moyen d'un filtre médical ou analogue, typiquement agencé en amont de la turbine, de manière à filtrer l'air aspiré par celle-ci et le débarrasser des poussières ou autres impuretés aérosols qu'il contient.

Le circuit de gaz peut comprendre une branche inspiratoire uniquement, c'est-à-dire être de type simple branche, ou alors comprendre une branche inspiratoire et une branche expiratoire, c'est-à-dire être de type à double branche.

L'évacuation des gaz riches en CO₂ expirés par le patient peut se faire au travers d'une valve expiratoire, par exemple agencée sur la branche expiratoire et/ou à proximité du patient. On parle alors de ventilation sans fuite intentionnelle, ou de ventilation sans fuite.

L'évacuation des gaz expirés peut, de manière alternative, se faire par un (ou des) orifice de fuite agencé au niveau d'un masque respiratoire ou analogue servant à dispenser le gaz au patient. On parle alors de ventilation à fuite intentionnelle ou de ventilation à fuite. L'expiration par un (ou des) orifices est typiquement associée à un circuit de type simple branche.

Usuellement, le (ou les) orifice est de taille insuffisante pour permettre une expiration confortable du patient, c'est-à-dire que la résistance à l'écoulement de l'orifice limite le débit naturel d'expiration du patient. C'est pourquoi les ventilateurs qui permettent la ventilation à fuite autorisent un débit inverse dans la branche inspiratoire, c'est-à-dire que le patient peut expirer librement à travers la branche inspiratoire en plus de pouvoir expirer à travers l'orifice.

Inversement, quand l'expiration se fait au travers d'une valve expiratoire, le ventilateur interdit usuellement tout débit inverse dans sa branche inspiratoire afin de limiter la potentielle ré-inhalation par le patient du gaz préalablement expiré.

Il existe des risques avec ces appareils qui peuvent liés à des surpressions gazeuses ou des défauts d'évacuation des gaz riches en CO₂ expirés par le patient.

Ainsi, pour un appareil avec branche expiratoire et valve expiratoire, en cas d'obstruction de la branche expiratoire ou de défaillance de la valve expiratoire, l'évacuation des gaz riches en CO₂ expirés par le patient peut être insuffisante jusqu'à engendrer un risque d'asphyxie chez le patient.

A l'inverse, l'inspiration du gaz peut aussi être difficile en cas de défaillance de la micro-soufflante ou d'obturation du circuit de gaz aux alentours de la micro-soufflante conduisant à un flux de gaz envoyé au patient insuffisant, engendrant alors aussi un risque d'asphyxie WO2017/149532A1 divulgue un appareil de ventilation artificielle comprenant un circuit de gaz principal pour acheminer un gaz respiratoire, ledit circuit de gaz principal comprenant une micro-soufflante, un circuit de bipasse venant se raccorder fluidiquement au circuit de gaz principal en un site de raccordement amont situé en amont de la micro-soufflante et en un site de raccordement aval, et une électrovanne de sécurité agencée sur le circuit de bipasse de manière à contrôler les flux gazeux dans ledit circuit de bipasse.

Au vu de cela, le problème qui se pose est de proposer un appareil de ventilation artificielle, c'est-à-dire un ventilateur médical amélioré conçu pour permettre à l'utilisateur d'opérer au choix une ventilation sans fuite ou à fuite intentionnelle et présentant un niveau de sécurité accru pour le patient, en particulier permettant :
- d'interdire ou d'autoriser un débit inverse dans la branche inspiratoire, selon que l'utilisateur souhaite opérer une ventilation à fuite ou sans fuite,
- pour une ventilation sans fuite, de réaliser une dépressurisation des voies aériennes du patient, en cas d'obstruction de tout ou partie des éléments de la branche expiratoire, de sorte d'éviter ou minimiser tout risque d'asphyxie chez le patient,
- et pour les deux types de ventilation, à fuite ou sans fuite, de permettre une inspiration aisée du patient en cas de dysfonctionnement de la micro-soufflante et de la partie de circuit de gaz située immédiatement en amont et/ou en aval de celle-ci, tout en assurant une filtration des gaz inspirés par le patient.

En d'autres termes, le but de l'invention est de proposer un appareil de ventilation amélioré permettant d'opérer au choix une ventilation à fuite ou sans fuite et configuré pour permettre notamment une dépressurisation des voies aériennes du patient et une évacuation des gaz riches en CO₂, en cas de défaillance de la branche expiratoire, y compris de la valve expiratoire, tout en assurant par ailleurs une étanchéité du circuit pneumatique en ventilation normale, c'est-à-dire en l'absence d'incident majeur, ou encore pour permettre à un patient d'inspirer du gaz filtré même lorsque la micro-soufflante est arrêtée.

L'invention concerne alors un appareil de ventilation artificielle, c'est-à-dire un ventilateur médical, comprenant un circuit de gaz principal pour acheminer un gaz respiratoire, ledit circuit de gaz principal comprenant une micro-soufflante et un dispositif anti-retour agencé en aval de la micro-soufflante, caractérisé en ce qu'il comprend en outre :
- un circuit de bipasse venant se raccorder fluidiquement au circuit de gaz principal en un site de raccordement amont situé en amont de la micro-soufflante et en un site de raccordement aval situé en aval du dispositif anti-retour, et
- une électrovanne de sécurité agencée sur le circuit de bipasse de manière à contrôler les flux gazeux dans ledit circuit de bipasse, ladite électrovanne de sécurité étant commandée par des moyens de pilotage comprenant une carte électronique à microprocesseur.

Selon le cas, l'appareil de ventilation artificielle de l'invention peut comprendre l'une ou plusieurs des caractéristiques techniques suivantes :
- le circuit de gaz principal comprend une portion aval formant une branche inspiratoire.
- la branche inspiratoire est en communication fluidique avec une interface respiratoire, de préférence un masque respiratoire ou une sonde d'intubation trachéale ou analogue.
- la branche inspiratoire comprend un tuyau flexible.
- il comprend en outre une branche expiratoire, en communication fluidique avec le circuit de gaz principal, comprenant une valve expiratoire commandée par un organe de commande de valve expiratoire. Ceci correspond notamment à une ventilation sans fuite.
- l'ouverture ou la fermeture de la valve expiratoire sont contrôlées par l'organe de commande de valve expiratoire via une ligne de pilotage pneumatique venant se raccorder fluidiquement au circuit de gaz principal, en un piquage situé entre la micro-soufflante et le dispositif anti-retour.
- la valve expiratoire permet d'évacuer les gaz expirés par le patient, c'est-à-dire les gaz expirés riches en CO₂, pendant les phases expiratoires, en fonctionnement normal.
- l'organe de commande de valve expiratoire comprend une (ou plusieurs) électrovanne de commande coopérant avec un orifice d'échappement ou un actionneur électromécanique.
- l'électrovanne de sécurité est commandée ou configurée pour s'ouvrir en cas de détection d'un dysfonctionnement de la branche expiratoire, de la valve expiratoire, de l'organe de commande de valve expiratoire ou de la micro-soufflante. Dans ce cas, l'électrovanne de sécurité est normalement fermée, c'est-à-dire en fonctionnement normal, i.e. en l'absence de tout dysfonctionnement.
- l'électrovanne de sécurité commandée ou configurée pour s'ouvrir en cas de détection d'un dysfonctionnement est de type tout ou rien.
- l'électrovanne de sécurité est commandée ou configurée pour s'ouvrir en cas d'obturation partielle ou totale, de la branche expiratoire.
- l'électrovanne de sécurité est commandée ou configurée pour s'ouvrir en cas de non-fonctionnement, i.e. d'arrêt, de la micro-soufflante.
- l'électrovanne de sécurité est commandée ou configurée pour s'ouvrir en cas de non-ouverture de la valve expiratoire et/ou de défaillance de l'organe de commande de valve expiratoire,
- le circuit de gaz principal est en communication fluidique avec une interface respiratoire, avantageusement un masque respiratoire, comprenant un (ou plusieurs) orifice de fuite, de manière à opérer une ventilation à fuite, et l'électrovanne de sécurité est commandée ou configurée pour soit s'ouvrir en cas de détection d'une expiration du patient, en particulier un début de phase expiratoire ou une fin de phase inspiratoire, soit se fermer en cas de détection d'une inspiration du patient, en particulier un début de phase inspiratoire.
- le circuit de gaz principal est en communication fluidique avec un masque respiratoire comprenant un (ou plusieurs) orifice de fuite agencé(s) dans le corps de masque ou dans un raccord tubulaire raccordé fluidiquement audit corps de masque.
- alternativement, notamment en cas de ventilation à fuite, l'électrovanne de sécurité est de type proportionnel, c'est-à-dire que la section de passage du gaz au travers de l'électrovanne varie entre plusieurs positions intermédiaires comprises entre une position d'obturation totale interdisant tout passage de gaz (i.e. totalement fermé) et une position d'ouverture maximale,
- l'électrovanne de sécurité est commandée ou configurée dans une première position Pe en cas de détection d'une expiration du patient, en particulier un début de phase expiratoire ou une fin de phase inspiratoire, et dans une seconde position Pi en cas de détection d'une inspiration du patient, en particulier un début de phase inspiratoire, telles que la section de passage de gaz dans la seconde position Pi soit inférieure à la section de passage de gaz dans la première position Pe.
- Selon l'invention, l'électrovanne de sécurité est commandée par des moyens de pilotage, de préférence,
- en l'absence d'alimentation électrique, l'électrovanne de sécurité reste dans sa dernière position ou adopte une position prédéterminée, ouverte ou fermée, préférentiellement une position ouverte.
- il comprend par ailleurs un (ou plusieurs) capteur de pression agencé de manière à mesurer la pression gazeuse dans le circuit de gaz principal.
- le capteur de pression est agencé en aval du site de raccordement aval.
- le capteur de pression est relié aux moyens de pilotage pour contrôler l'électrovanne de sécurité.
- le circuit de gaz principal comprend en outre des moyens de filtration de gaz agencés en amont du site de raccordement amont et/ou en aval du site de raccordement aval.
- les moyens de filtration de gaz comprennent un (ou plusieurs) filtre(s) adaptés à une élimination des poussières, pollens, bactéries et/ou autres microorganismes, par exemple des filtres de type HEPA.
- il comprend au moins un capteur de débit agencé de manière à mesurer le débit gazeux dans le circuit de gaz principal, les mesures opérées par le capteur de débit permettant notamment de détecter les phases inspiratoires et/ou expiratoires du patient et donc de piloter l'électrovanne de sécurité, en particulier pendant une ventilation à fuite.
- de préférence, un premier capteur de débit est agencé au voisinage du capteur de pression et un second capteur de débit est agencé à proximité du patient ou sur la branche expiratoire, notamment pour opérer une détection de fuites non intentionnelles ou alarmer en cas d'hypoventilation du patient, lors de la mise en œuvre d'une ventilation sans fuite.
- Selon l'invention, les moyens de pilotage comprennent une carte électronique à microprocesseur, de préférence à microcontrôleur, configuré pour agir sur l'électrovanne de sécurité en réponse à un signal provenant du capteur de pression et/ou de débit.
- les moyens de pilotage sont configurés pour agir sur l'électrovanne de sécurité en cas de détection d'une pression anormale dans le circuit principal de gaz, en particulier dans la branche inspiratoire. Par exemple, une pression anormale peut correspondre à une valeur de pression supérieure à un seuil de pression maximale donné.
- les moyens de pilotage sont configurés pour mesurer la vitesse de rotation de la turbine, par exemple au travers de la mesure d'un signal tachymétrique.
- les moyens de pilotage sont configurés pour agir sur l'électrovanne de sécurité en cas de détection d'un fonctionnement anormal de la turbine, par exemple une vitesse de rotation trop faible (i.e. inférieure à un seuil de vitesse donné) et/ou une pression inférieure à un seuil de pression donné et/ou un débit inférieur à un seuil de débit donné.
- les moyens de pilotage comprennent une carte électronique à microprocesseur et des moyens d'horloge internes, par exemple un oscillateur à quartz ou électronique, configurés pour agir sur l'électrovanne de sécurité à des intervalles de temps prédéfinis de manière à autoriser ou interdire, de manière séquentielle, le passage de gaz au travers de l'électrovanne de sécurité.
- le circuit de gaz principal comprend une entrée de gaz, i.e. un orifice ou analogue, en communication fluidique avec l'atmosphère ambiante ou une canalisation de gaz, de préférence avec l'atmosphère ambiante.
- de l'air atmosphérique est aspiré par la micro-soufflante via l'entrée de gaz du circuit de gaz principal.
- le dispositif anti-retour comprend un clapet anti-retour de type parapluie ou de type bec de canard.
- la micro-soufflante est commandée par les moyens de pilotage pour délivrer du gaz respiratoire, en particulier de l'air ou de l'air enrichi en oxygène.
- le circuit de gaz principal comprend une entrée d'oxygène, par exemple un raccord ou analogue, permettant le raccordement d'une source d'oxygène et l'adjonction d'O₂ dans le circuit de gaz principal, de préférence l'entrée d'oxygène est située en amont de la micro-soufflante.
- il comprend une branche inspiratoire et une branche expiratoire reliées l'une à l'autre par une pièce de raccordement, en particulier une pièce en Y.

L'invention va maintenant être mieux comprise grâce à la description détaillée suivante, faite à titre illustratif mais non limitatif, en référence aux figures annexées parmi lesquelles :
- la Figure 1 est un schéma du circuit pneumatique d'un appareil de ventilation artificielle à branches inspiratoire et expiratoire selon la présente invention, pour mise en œuvre d'une ventilation sans fuite,
- la Figure 2 schématise le fonctionnement du circuit pneumatique de la Figure 1, lors d'une inspiration en fonctionnement normal,
- la Figure 3 schématise le fonctionnement du circuit pneumatique de la Figure 1, lors d'une expiration en fonctionnement normal,
- la Figure 4 schématise le fonctionnement du circuit pneumatique de la Figure 1, lors d'une expiration en cas d'obstruction du circuit,
- la Figure 5 schématise le fonctionnement du circuit pneumatique de la Figure 1, lors d'une inspiration en cas d'arrêt intempestif de la turbine,
- la Figure 6 schématise le fonctionnement du circuit pneumatique d'un appareil de ventilation artificielle à branche respiratoire unique, pour mise en œuvre d'une ventilation avec fuite intentionnelle, selon la présente invention, pendant une phase d'inspiration de gaz par le patient, et
- la Figure 7 schématise le fonctionnement du circuit pneumatique de l'appareil de ventilation de la Figure 6, pendant une phase d'expiration de gaz par le patient.

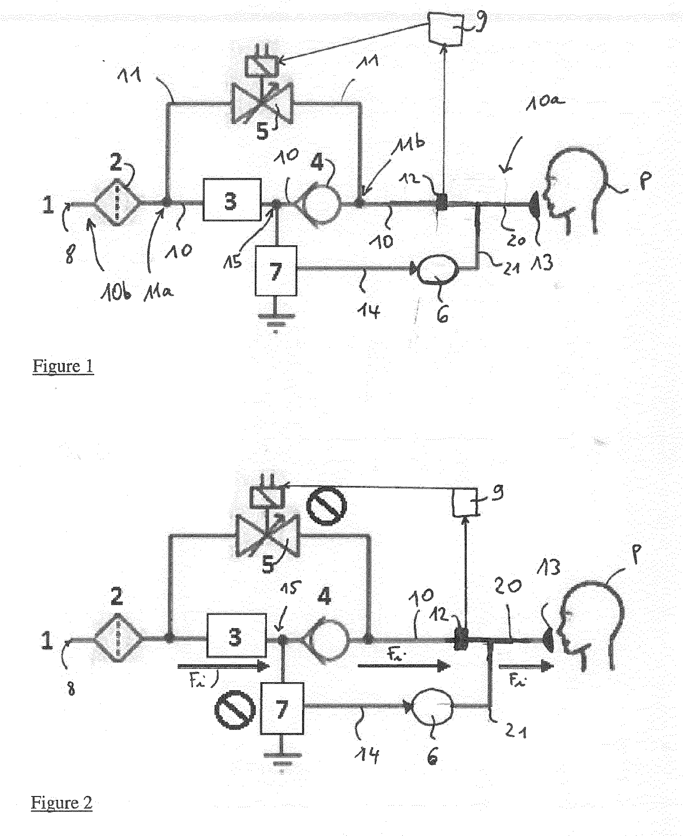

La Figure 1 est un schéma du circuit pneumatique d'un appareil de ventilation artificielle ou ventilateur médical selon la présente invention à branche inspiratoire 20 et branche expiratoire 21, adapté à la mise en œuvre d'une ventilation sans fuite.

Il comprend un circuit de gaz principal 10 pour acheminer un gaz respiratoire, par exemple de l'air ou de l'air enrichi en oxygène, jusqu'à un patient P.

Le circuit de gaz principal 10 comprend un ou des passages pour le gaz, tels que des conduits, tubes ou analogues. La portion aval 10a du circuit de gaz principal 10 forme tout ou partie de la branche inspiratoire 20, laquelle est en communication fluidique avec une interface respiratoire 13, tel un masque respiratoire ou une sonde d'intubation trachéale.

Il est également prévu, sur le circuit de gaz principal 10, une micro-soufflante 3, encore appelée turbine ou compresseur, et un dispositif anti-retour 4, tel un clapet anti-retour, agencé en aval de la micro-soufflante 3. Le clapet anti-retour 4 est préférentiellement de type parapluie ou de type bec de canard.

La portion amont 10b du circuit de gaz principal 10 est en communication fluidique, via un orifice d'entrée 8, avec une source de gaz 1, typiquement l'air atmosphérique, de sorte d'alimenter la micro-soufflante 3 en air atmosphérique.

Afin de garantir un gaz sans impuretés aérosols, par exemple sans poussière, pollens, microorganismes ou autres polluants, le circuit de gaz principal 10 est préférentiellement équipé de moyens de filtration de gaz 2, tel un (ou plusieurs) filtre, par exemple de type HEPA, agencés sur le trajet du gaz au sein du circuit de gaz principal 10, en particulier en amont du site de raccordement amont 11a et de la micro-soufflante 3. Ainsi, la qualité de l'air fourni au patient P est plus élevée que celle de l'air ambiant puisque le filtre retient tout ou partie des impuretés aérosols qui peuvent s'y trouver, tel que poussières, pollens, microorganismes...

On peut également prévoir, en amont de la micro-soufflante 3, une entrée d'oxygène (non montré sur les Figures), par exemple un raccord ou analogue, permettant le raccordement d'une source d'oxygène et l'adjonction d'O₂ dans le circuit de gaz principal, donc dans l'air aspiré par la micro-soufflante 3.

Par ailleurs, le circuit de gaz principal 10 comprend aussi une valve expiratoire 6 agencée sur la branche expiratoire 21, laquelle permet, en fonctionnement normal, d'évacuer les gaz riches en CO₂ expirés par le patient P, pendant les phases expiratoires.

L'ouverture et la fermeture de cette valve expiratoire 6 se fait via une ligne de pilotage pneumatique 14 qui vient se raccorder fluidiquement au circuit de gaz principal 10, en un piquage 15 situé entre la micro-soufflante 3 et le dispositif anti-retour 4, de manière à être alimentée, en fonctionnement normal, en gaz sous pression provenant de la micro-soufflante 3. Une telle ligne de pilotage pneumatique 14 est classique.

Pendant les phases inspiratoires du patient P, le gaz sous pression traversant la micro-soufflante 3 pénètre dans la ligne de pilotage pneumatique 14 via le piquage 15 et va alors agir sur la valve expiratoire 6 pour la maintenir en position fermée, par exemple par actionnement d'une membrane ou d'un ballonnet.

A l'inverse, pendant les phases expiratoires du patient P, la valve expiratoire 6 passe en position ouverte ou partiellement ouverte pour évacuer les gaz expirés par le patient P. Le fonctionnement de la valve expiratoire 6, c'est-à-dire son ouverture et sa fermeture pneumatiques, est donc déterminé par le niveau de pressurisation de la ligne de pilotage pneumatique 14.

La pression dans la ligne de pilotage pneumatique 14 est notamment contrôlée par un dispositif ou organe de commande 7 de valve expiratoire pneumatique 6, par exemple une ou des (électro)vannes, agencé sur la ligne de pilotage pneumatique 14. L'organe de commande peut également coopérer avec un (ou des) orifice permettant de relier fluidiquement la ligne de commande 14 à l'atmosphère.

Selon la présente invention, l'appareil comprend en outre un circuit de bipasse 11, à savoir une canalisation, une ligne ou un passage de gaz, équipé d'une électrovanne de sécurité 5, comme montré sur les Figures.

Le circuit de bipasse 11 vient se raccorder fluidiquement au circuit de gaz principal 10 en un site de raccordement amont 11a situé en amont de la micro-soufflante 3 et en un site de raccordement aval 11b situé en aval du dispositif anti-retour 4. Le circuit de bipasse 11 permet donc de bipasser pneumatiquement la micro-soufflante 3.

Une électrovanne de sécurité 5 est agencée sur le circuit de bipasse 11, par exemple une électrovanne « tout ou rien » ou une électrovanne proportionnelle, laquelle permet de contrôler les flux gazeux dans le circuit de bipasse 11, c'est-à-dire la circulation du gaz au sein dudit circuit de bipasse 11 du site de raccordement amont 1 la au site aval 11b, et inversement.

L'électrovanne de sécurité 5 est normalement fermée, en fonctionnement normal, c'est-à-dire en l'absence de tout dysfonctionnement. Plus précisément, l'électrovanne de sécurité 5 est configurée et/ou commandée par des moyens de pilotage 9 pour s'ouvrir en cas de détection d'un dysfonctionnement de la branche expiratoire 21, de la valve expiratoire 6, de l'organe de commande 7 de valve expiratoire ou de la micro-soufflante 3, par exemple en cas d'obturation de la branche expiratoire 21 ou en cas de défaillance de la valve expiratoire 6, ou encore d'arrêt intempestif de la micro-soufflante 3.

L'électrovanne de sécurité 5 est commandée par des moyens de pilotage 9, telle une carte électronique à microprocesseur, préférentiellement à microcontrôleur, en fonction de signaux provenant d'un capteur de pression 12 dont la prise de pression est agencée sur le circuit de gaz principal 10 de manière à pouvoir y détecter toute surpression causée par un dysfonctionnement. Le capteur de pression 12 est agencé dans la portion aval 10a du circuit de gaz principal 10, par exemple au niveau de la branche inspiratoire 20 ou de la branche expiratoire 21.

Selon le mode de réalisation choisi, l'électrovanne de sécurité 5 peut être de type tout ou rien ou de type proportionnel de manière à pouvoir interdire complètement ou seulement partiellement le passage de gaz dans le circuit de bipasse 11.

En cas de détection d'un dysfonctionnement engendrant par exemple une surpression dans le circuit principal 10, due par exemple un défaut d'ouverture de la valve expiratoire 6, les moyens de pilotage 9 commandent l'électrovanne de sécurité 5 de manière à autoriser le passage de gaz, en particulier des gaz expirés par le patient, dans le circuit de bipasse 11, lesquels peuvent alors être évacués à l'atmosphère 1 via l'orifice 8.

De même, en cas d'arrêt intempestif de la micro-soufflante 3, l'électrovanne de sécurité 5 passe en position ouverte et le patient peut respirer au travers de la ligne de bipasse 11, c'est-à-dire que les échanges gazeux d'air entrant et de gaz riches en CO₂ rejetés à l'atmosphère se font via l'orifice 8. Le gaz inspiré par le patient est alors filtré au travers du (ou des) filtre 2.

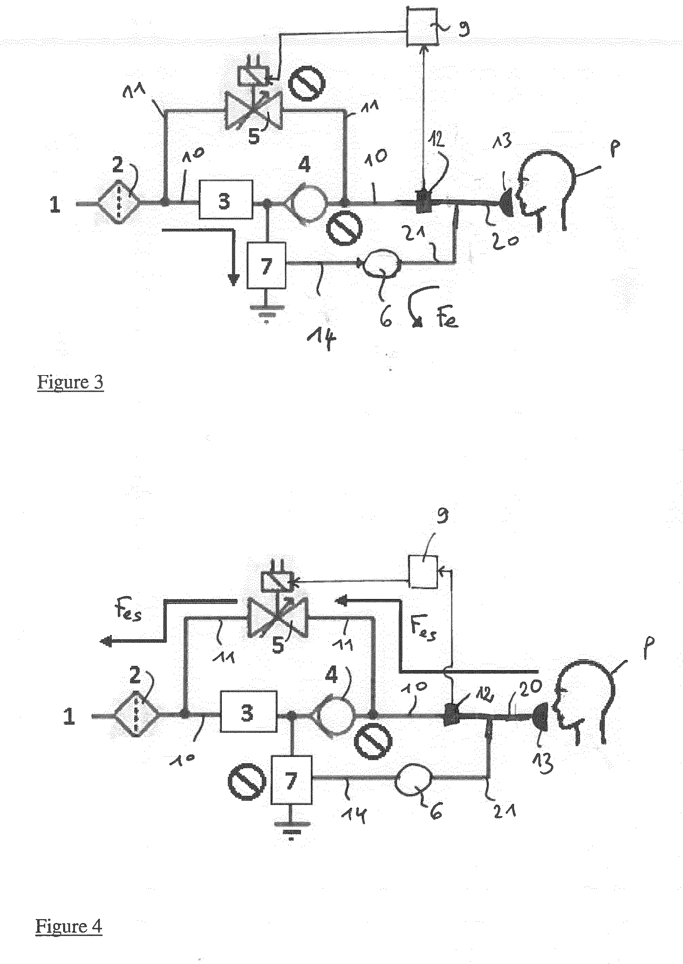

Les Figures 2 et 3 illustrent ainsi la dynamique d'une inspiration et d'une expiration en ventilation sans fuite, au travers du circuit pneumatique de la Figure 1, lors d'un fonctionnement normal, c'est-à-dire en l'absence de dysfonctionnement.

Comme on le voit sur ces Figures 2 et 3, en ventilation normale, c'est-à-dire pendant les phases d'expiration (Fig. 3) ou d'inspiration (Fig. 2) du patient P, l'électrovanne de sécurité 5 est fermée et le circuit pneumatique, c'est-à-dire le circuit de gaz principal 10, est donc étanche. Le patient P est alors alimenté en air provenant de l'atmosphère 1, via la micro-soufflante 3.

Pendant les phases d'inspiration (Fig. 2), le gaz traverse donc successivement la micro-soufflante 3 et le dispositif anti-retour 4, en particulier un clapet, et chemine dans le circuit 10 et la branche inspiratoire 20 en direction du patient P (flèche Fi en Fig. 2) et, en parallèle, une partie du gaz va aller fermer la valve expiratoire 6 via la ligne de pilotage pneumatique 14, comme expliqué ci-avant.

A l'inverse, pendant les phases d'expiration (Fig. 3), la micro-soufflante 3 ne délivre plus (ou très peu) de gaz et l'évacuation vers l'atmosphère des gaz riches en CO₂ expirés par le patient P se fait alors via la branche expiratoire 21 et au travers de la valve expiratoire 6 qui est alors en position ouverte de sorte de faire communiquer fluidiquement le circuit de gaz principal 10 avec l'extérieur, c'est-à-dire l'atmosphère ambiante. En effet, la pression du flux de gaz dans la ligne de pilotage pneumatique 14 est alors très réduite ou même nulle, par exemple suite à une commande de l'organe de commande 7 de la valve expiratoire 6 pneumatique par les moyens de pilotage 9, ce qui ouvre alors la valve expiratoire 6 et permet aux gaz riches en CO₂ expirés par le patient P de s'échapper à l'atmosphère (flèche Fe en Fig. 3).

Selon un mode de réalisation alternatif (non montré), l'ouverture ou fermeture de la valve expiratoire 6 peut aussi être géré de façon électromagnétique par l'organe de commande 7 plutôt que de façon pneumatique.

D'une façon générale, on comprend donc qu'en fonctionnement normal, l'électrovanne 5 est toujours fermée que ce soit pendant l'inspiration ou pendant l'expiration du patient P, puisque les moyens de pilotage 9 ne commandent pas son ouverture tant qu'ils ne détectent pas à une surpression anormale dans le circuit de gaz principal 10 causée par un dysfonctionnement, par exemple une obstruction de la branche expiratoire 21, une défaillance de la valve expiratoire 6 ou encore un arrêt de la micro-soufflante 3.

La Figure 4 schématise, quant à elle, la dynamique d'une expiration de gaz en cas de dysfonctionnement, par exemple une obstruction la branche expiratoire 21 due à un écrasement de tuyaux ou à des sécrétions ou de la condensation venant boucher le passage de gaz ou encore à un défaut de l'organe de commande 7 empêchant la dépressurisation de la ligne de pilotage 14, donc l'ouverture de la valve expiratoire 6.

Ce dysfonctionnement engendre une surpression gazeuse dans le circuit 10 qui est détectée par le capteur de pression 12 dont la prise de pression est agencée dans le circuit 10, de préférence en aval 10a du circuit 10, avantageusement à proximité du patient P. Le signal de mesure émanant du capteur 12 est transmis électriquement aux moyens de pilotage 9 qui pilotent alors l'électrovanne de sécurité 5 pour en provoquer l'ouverture et autoriser les circulations de gaz dans la branche de bipasse 11.

Dans ce cas, pendant les phases expiratoires du patient P, l'électrovanne de sécurité 5 est maintenue ouverte de sorte que le gaz contenu dans les voies aériennes du patient puisse être expiré successivement au travers de la partie aval 10a du circuit 10 comprenant la branche inspiratoire 20, de la ligne de bipasse 11, de l'électrovanne de sécurité 5, de la portion amont 10b du circuit 10 comprenant le filtre 2 ou analogue (flèches Fes en Fig. 4), puis de l'orifice 8 qui est normalement l'entrée de gaz alimentant la micro-soufflante 3 en air atmosphérique.

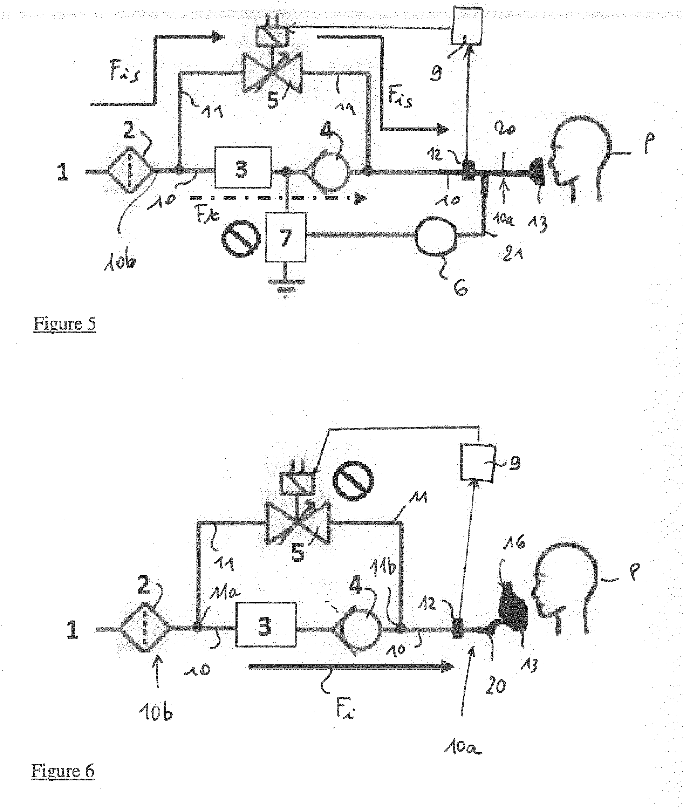

De manière analogue, comme illustré en Figure 5, pendant les phases inspiratoires du patient P, en cas d'arrêt intempestif de la micro-soufflante 3 par exemple, l'électrovanne de sécurité 5 est commandée par les moyens de pilotage 9 pour être maintenue ouverte de sorte que le patient P puisse inspirer de l'air filtré qui entre par l'orifice 8 et chemine ensuite (flèches Fis en Fig. 5) entre la portion amont 10b et la portion aval 10a comprenant la branche inspiratoire 20, au travers de la ligne de bipasse 11 et de l'électrovanne de sécurité 5. Avantageusement, une partie de l'air inspiré peut aussi passer directement au travers de la micro-soufflante 3 (flèche Ft). Le fait de pouvoir, dans ce cas, inspirer au travers à la fois de la micro-soufflante 3 et de la ligne de bipasse 11 présente l'avantage de réduire l'effort inspiratoire du patient P.

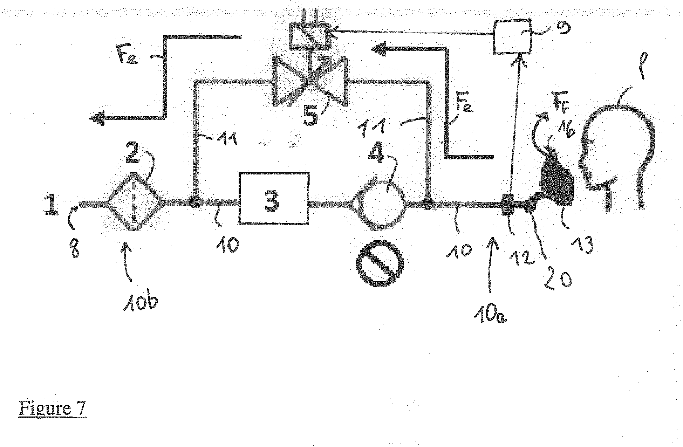

Par ailleurs, les Figures 6 et 7 schématisent le fonctionnement d'un autre mode de réalisation du circuit pneumatique d'un appareil de ventilation artificielle selon la présente invention, à savoir ici un circuit pneumatique à branche respiratoire unique conçu pour réaliser une ventilation à fuite intentionnelle.

La Figure 6 schématise la phase d'inspiration, alors que la Figure 7 schématise la phase d'expiration.

Le circuit pneumatique des Figures 6 et 7 est similaire à celui des Figures 1 à 5, à l'exception du fait que la branche expiratoire, la valve expiratoire, l'organe de commande de valve expiratoire et la ligne de pilotage pneumatique ont été supprimés puisque, dans ce mode de réalisation, l'évacuation des gaz expirés par le patient se fait, en fonctionnement normal, via un (ou plusieurs) orifice de fuite 16 agencé sur l'interface respiratoire 13, avantageusement un masque respiratoire. L'orifice de fuite 16 peut être agencé dans le corps de masque ou dans un raccord tubulaire connecté fluidiquement au corps de masque. Alternativement (non représenté en Figures 6 et 7), l'organe de commande de valve expiratoire et tout ou partie de la ligne de pilotage pneumatique peuvent être conservés mais commandée de sorte à ce qu'aucun débit ni aucune pression ne soit présente dans la ligne de pilotage pneumatique.

Dans ce cas, l'électrovanne de sécurité 5 est configurée ou commandée par les moyens de pilotage 9 pour :
- soit s'ouvrir en cas de détection par le capteur de pression 12, d'une expiration du patient, en particulier un début de phase expiratoire ou une fin de phase inspiratoire, et/ou
- soit se fermer en cas de détection par le capteur de pression 12, d'une inspiration du patient, en particulier un début de phase inspiratoire.

L'électrovanne de sécurité 5 est préférentiellement de type proportionnel, c'est-à-dire que la section de passage du gaz au travers de l'électrovanne 5 varie entre plusieurs positions intermédiaires comprises entre une position d'obturation totale interdisant tout passage de gaz (i.e. passage totalement fermé) et une position d'ouverture maximale.

En d'autres termes, en phase inspiratoire (Fig. 6), l'électrovanne 5 se ferme totalement ou partiellement et le débit passe de la source 1 de gaz, typiquement l'atmosphère ambiante, au patient P au travers de la micro-soufflante 3 et de la branche inspiratoire 20 (Flèche Fi sur Fig. 6).

Par ailleurs, en phase expiratoire (Fig. 7), l'électrovanne 5 s'ouvre totalement ou partiellement et le gaz contenu dans les voies aériennes du patient P est évacué, d'une part, par l'orifice de fuite 16 du masque 13 (Flèche Ff sur Fig. 7) et, d'autre part, au travers de la ligne de bipasse 11, de l'électrovanne de sécurité 5, de la portion amont 10b du circuit 10 et l'orifice 8 (Flèches Fe sur Fig. 7).

D'une façon générale, dans le cadre de la présente invention et dans les modes de réalisation représentés sur les Figures 1-7, la détection des dysfonctionnements (obstruction, bouchage, défaut turbine...) ou des phases expiratoires ou inspiratoires du patient P, est opérée par suivi de la pression au moyen du capteur 12 de pression et détection de toute pression anormale, par exemple un niveau de pression supérieur à une valeur-seuil.

Optionnellement, au moins un capteur de débit (non montré sur les Figures 1-7) est agencé sur le circuit de gaz principal de gaz 10 de manière à mesurer le débit gazeux dans le circuit de gaz principal 10, de préférence en aval du site de raccordement aval 11b, ledit capteur de débit étant relié aux moyens de pilotage 9.

De préférence, on prévoit un premier capteur de débit agencé au voisinage du capteur de pression 12 et un autre capteur de débit agencé à proximité du patient, par exemple sur la branche expiratoire d'un appareil selon les Figures 1-5.

Le capteur de débit est par exemple un capteur à fil chaud, ou un capteur de pression différentielle dont les deux raccordements sont situés de part et d'autre d'un élément qui résiste à l'écoulement, par exemple un passage de gaz dont la section est réduite.

Les mesures opérées par le (ou les) capteur de débit permettent notamment de détecter:
- les phases expiratoires du patient et donc de piloter l'électrovanne 5 dans le mode de ventilation à fuite, c'est-à-dire le mode de réalisation des Figures 6 et 7, ou
- d'éventuelles fuites non intentionnelles ou une hypoventilation du patient dans le mode de ventilation sans fuite, c'est-à-dire le mode de réalisation des Figures 1 à 5.

Optionnellement, les moyens de pilotage 9 sont reliés à la micro-soufflante 3 de sorte de mesurer sa vitesse de rotation, par exemple par la lecture d'un signal tachymétrique généré par la micro-soufflante. Préférentiellement, ce signal est généré par une (ou des) sonde à effet Hall qui génère une impulsion électrique lorsque l'hélice de la micro-soufflante et/ou le moteur électrique de la micro-soufflante, est dans une (ou des) position angulaire particulière. Les moyens de pilotage 9 sont connectés électriquement à la sonde de sorte que la mesure de la fréquence de l'impulsion électrique par lesdits moyens de pilotage permet de mesurer la vitesse de rotation de la micro-soufflante 3.

La mesure de vitesse ainsi opérée permet notamment de détecter un dysfonctionnement de la micro-soufflante 3, par exemple une vitesse de micro-soufflante insuffisante, c'est-à-dire inférieure à un seuil de vitesse minimale donné. En cas d'un tel dysfonctionnement, les moyens de pilotage 9 pilotent ou configurent la vanne de sécurité 5 en position ouverte, permettant l'évacuation des gaz riches en CO₂ expirés par le patient.

L'appareil de ventilation artificielle selon l'invention offre un niveau de sécurité accru pour le patient, que ce soit en ventilation sans fuite ou en ventilation à fuite, notamment en facilitant l'évacuation des gaz riches en CO₂ expirés par le patient.

## Revendications

1. Appareil de ventilation artificielle comprenant un circuit de gaz principal (10) pour acheminer un gaz respiratoire, ledit circuit de gaz principal (10) comprenant une micro-soufflante (3) et un dispositif anti-retour (4) agencé en aval de la micro-soufflante (3), ledit appareil comprenant en outre :
- un circuit de bipasse (11) venant se raccorder fluidiquement au circuit de gaz principal (10) en un site de raccordement amont (11a) situé en amont de la micro-soufflante (3) et en un site de raccordement aval (11b) situé en aval du dispositif anti-retour (4), et
- une électrovanne de sécurité (5) agencée sur le circuit de bipasse (11) de manière à contrôler les flux gazeux dans ledit circuit de bipasse (11), ladite électrovanne de sécurité (5) étant commandée par des moyens de pilotage (9) comprenant une carte électronique à microprocesseur

2. Appareil selon la revendication 1, **caractérisé en ce qu'**il comprend en outre une branche expiratoire (21) en communication fluidique avec le circuit de gaz principal (10), comprenant une valve expiratoire (6) commandée par un organe de commande (7) de valve expiratoire, l'électrovanne de sécurité (5) étant commandée ou configurée pour s'ouvrir en cas de détection d'un dysfonctionnement de la branche expiratoire (21), de la valve expiratoire (6), de l'organe de commande (7) de valve expiratoire ou de la micro-soufflante (3).

3. Appareil selon la revendication 2, **caractérisé en ce que** l'ouverture ou la fermeture de la valve expiratoire (6) sont contrôlées par l'organe de commande (7) de valve expiratoire via une ligne de pilotage pneumatique (14) venant se raccorder fluidiquement au circuit de gaz principal (10), en un piquage (15) situé entre la micro-soufflante (3) et le dispositif anti-retour (4).

4. Appareil selon la revendication 1, **caractérisé en ce qu'**il comprend une interface respiratoire (13) comprenant un orifice de fuite (16), l'électrovanne de sécurité (5) étant commandée ou configurée pour s'ouvrir en cas de détection d'une expiration du patient ou se fermer en cas de détection d'une inspiration du patient.

5. Appareil selon l'une des revendications précédentes, **caractérisé en ce que** l'électrovanne de sécurité (5) est de type proportionnel.

6. Appareil selon la revendication 1, **caractérisé en ce qu'**il comprend un capteur de pression (12) agencé de manière à mesurer la pression gazeuse dans le circuit de gaz principal (10), en aval du site de raccordement aval (11b), ledit capteur de pression (12) étant relié aux moyens de pilotage (9) pour contrôler l'électrovanne de sécurité (5).

7. Appareil selon l'une des revendications précédentes, **caractérisé en ce que** le circuit de gaz principal (10) comprend en outre des moyens de filtration de gaz (2) agencés en amont du site de raccordement amont (11a).

8. Appareil selon la revendication 1, **caractérisé en ce que** les moyens de pilotage (9) comprennent une carte électronique à microcontrôleur, configuré pour agir sur l'électrovanne de sécurité (5) en réponse à un signal provenant du capteur de pression et/ou d'un capteur de débit et/ou d'un capteur de vitesse.

9. Appareil selon l'une des revendications 1 à 8, **caractérisé en ce que** le circuit de gaz principal (10) comprend une branche inspiratoire (20) située à une extrémité aval (10a) dudit circuit de gaz principal (10).

## Patentansprüche

1. Vorrichtung zur künstlichen Beatmung, umfassend einen Hauptgaskreislauf (10) zum Leiten eines Atemgases, wobei der Hauptgaskreislauf (10) ein Mikrogebläse (3) und eine stromabwärts des Mikrogebläses (3) angeordnete Rückschlagvorrichtung (4) umfasst, wobei die Vorrichtung weiter umfasst:
- einen Umgehungskreislauf (11), der fluidisch mit dem Hauptgaskreislauf (10) an einer stromaufwärtigen Verbindungsstelle (11a), die sich stromaufwärts des Mikrogebläses (3) befindet, und an einer stromabwärtigen Verbindungsstelle (11b), die sich stromabwärts der Rückschlagvorrichtung (4) befindet, verbunden ist, und
- ein Sicherheitsmagnetventil (5), das auf dem Umgehungskreislauf (11) so angeordnet ist, dass es die Gasströmungen in dem Umgehungskreislauf (11) steuert, wobei das Sicherheitsmagnetventil (5) durch Steuermittel (9) gesteuert wird, die eine elektronische Mikroprozessorkarte umfassen.

2. Vorrichtung nach Anspruch 1, **dadurch gekennzeichnet, dass** sie weiter einen Ausatmungszweig (21) in Fluidverbindung mit dem Hauptgaskreislauf (10) umfasst, der ein Ausatmungsventil (6) umfasst, das durch ein Ausatmungsventilsteuerorgan (7) gesteuert wird, wobei das Sicherheitsmagnetventil (5) so gesteuert oder konfiguriert ist, dass es sich bei Erkennung einer Fehlfunktion des Ausatmungszweiges (21), des Ausatmungsventils (6), des Ausatmungsventilsteuerorgans (7) oder des Mikrogebläses (3) öffnet.

3. Vorrichtung nach Anspruch 2, **dadurch gekennzeichnet, dass** das Öffnen oder das Schließen des Ausatmungsventils (6) durch das Ausatmungsventilsteuerorgan (7) über eine pneumatische Steuerleitung (14) gesteuert wird, die fluidisch mit dem Hauptgaskreislauf (10) an einer Abzweigung (15) verbunden ist, die sich zwischen dem Mikrogebläse (3) und der Rückschlagvorrichtung (4) befindet.

4. Vorrichtung nach Anspruch 1, **dadurch gekennzeichnet, dass** sie eine Atmungsschnittstelle (13) mit einer Leckageöffnung (16) umfasst, wobei das Sicherheitsmagnetventil (5) so gesteuert oder konfiguriert ist, dass es sich bei Erkennung einer Ausatmung des Patienten öffnet oder bei Erkennung einer Einatmung des Patienten schließt.

5. Vorrichtung nach einem der vorstehenden Ansprüche, **dadurch gekennzeichnet, dass** es sich bei dem Sicherheitsmagnetventil (5) um eines vom Proportionaltyp handelt.

6. Vorrichtung nach Anspruch 1, **dadurch gekennzeichnet, dass** sie einen Drucksensor (12) umfasst, der so angeordnet ist, dass er den Gasdruck im Hauptgaskreislauf (10) stromabwärts der stromabwärtigen Verbindungsstelle (11b) misst, wobei der Drucksensor (12) mit Steuermitteln (9) zur Steuerung des Sicherheitsmagnetventils (5) verbunden ist.

7. Vorrichtung nach einem der vorstehenden Ansprüche, **dadurch gekennzeichnet, dass** der Hauptgaskreislauf (10) weiter Gasfiltermittel (2) umfasst, die stromaufwärts der stromaufwärtigen Verbindungsstelle (11a) angeordnet sind.

8. Vorrichtung nach Anspruch 1, **dadurch gekennzeichnet, dass** die Steuermittel (9) eine elektronische Mikrocontrollerkarte umfassen, die konfiguriert ist, um auf das Sicherheitsmagnetventil (5) als Reaktion auf ein Signal von dem Drucksensor und/oder einem Durchsatzsensor und/oder einem Geschwindigkeitssensor einzuwirken.

9. Vorrichtung nach einem der Ansprüche 1 bis 8, **dadurch gekennzeichnet, dass** der Hauptgaskreislauf (10) einen Einatmungszweig (20) umfasst, der an einem stromabwärtigen Ende (10a) des Hauptgaskreislaufs (10) angeordnet ist.

## Claims

1. Artificial ventilation apparatus comprising a main gas circuit (10) to convey a breathing gas, said main gas circuit (10) comprising a micro blower (3) and a non-return device (4) arranged downstream from the micro blower (3), said apparatus further comprising:
- a bypass circuit (11) fluidically connected to the main gas circuit (10) in an upstream connection site (11a) situated upstream from the micro blower (3) and in a downstream connection site (11b) situated downstream from the non-return device (4), and
- a safety solenoid valve (5) arranged on the bypass circuit (11) so as to control the gas streams in said bypass circuit (11), said safety solenoid valve (5) being controlled by control means (9) further comprising a microprocessor electronic card.

2. Apparatus according to claim 1, **characterised in that** it further comprises an expiratory branch (21) in fluidic communication with the main gas circuit (10), comprising an expiratory valve (6) controlled by an expiratory valve control member (7), the safety solenoid valve (5) being controlled or configured to be opened in case of detecting a malfunctioning of the expiratory branch (21), of the expiratory valve (6), of the expiratory valve control member (7) or the micro blower (3).

3. Apparatus according to claim 2, **characterised in that** the opening or the closing of the expiratory valve (6) are controlled by the expiratory valve control member (7) via a pneumatic control line (14) being fluidically connected to the main gas circuit (10), in a pitting (15) situated between the micro blower (3) and the non-return device (4).

4. Apparatus according to claim 1, **characterised in that** it comprises a breathing interface (13) comprising a leak orifice (16), the safety solenoid valve (5) being controlled or configured to be opened in case of detecting an exhalation of the patient or be closed in case of detecting an inhalation of the patient.

5. Apparatus according to one of the preceding claims, **characterised in that** the safety solenoid valve (5) is of the proportional type.

6. Apparatus according to claim 1, **characterised in that** it comprises a pressure sensor (12) arranged so as to measure the gas pressure in the main gas circuit (10), downstream from the downstream connection site (11b), said pressure sensor (12) being connected to the control means (9) to control the safety solenoid valve (5).

7. Apparatus according to one of the preceding claims, **characterised in that** the main gas circuit (10) further comprises gas filtration means (2) arranged upstream from the upstream connection site (11a).

8. Apparatus according to claim 1, **characterised in that** the control means (9) comprise a microcontroller electronic card, configured to act on the safety solenoid valve (5) in response to a signal coming from the pressure sensor and/or a flow sensor and/or a speed sensor.

9. Apparatus according to one of claims 1 to 8, **characterised in that** the main gas circuit (10) comprises an inspiratory branch (20) situated at a downstream end (10a) of said main gas circuit (10).
